(19) Europäisches Patentamt
European Patent Office
Office européen des brevets

(11) EP 3 944 806 A1

(12) EUROPÄISCHE PATENTANMELDUNG

(43) Veröffentlichungstag:
02.02.2022 Patentblatt 2022/05

(51) Internationale Patentklassifikation (IPC):
A61B 3/10 (2006.01)

(21) Anmeldenummer: 20188386.5

(52) Gemeinsame Patentklassifikation (CPC):
A61B 3/10

(22) Anmeldetag: 29.07.2020

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR
Benannte Erstreckungsstaaten:
BA ME
Benannte Validierungsstaaten:
KH MA MD TN

(71) Anmelder: Carl Zeiss Vision International GmbH
73430 Aalen (DE)

(72) Erfinder: Weimann, Claudius
73560 Böbingen (DE)

(74) Vertreter: Sticht, Andreas
Kraus & Weisert
Patentanwälte PartGmbB
Thomas-Wimmer-Ring 15
80539 München (DE)

(54) **VERFAHREN ZUM BESTIMMEN DES NAHPUNKTS, ZUM BESTIMMEN DER NAHPUNKTDISTANZ, ZUM BESTIMMEN EINES SPHÄRISCHEN BRECHWERTES SOWIE ZUM HERSTELLEN EINES BRILLENGLASES SOWIE ENTSPRECHENDE MOBILE ENDGERÄTE UND COMPUTERPROGRAMME**

(57) Es wird ein Verfahren zum Bestimmen eines Nahpunkts und einer Nahpunktdistanz (31) einer Person (30) bereitgestellt. Weitergehend werden auch Verfahren zum Bestimmen eines sphärischen Brechwertes durchgeführt. Dabei werden in einer Implementierung Sehzeichen auf einem mobilen Endgerät (10) angezeigt, und die Person (30) wird aufgefordert, das mobile Endgerät (10) zu dem Nahpunkt der Person (30) zu bewegen.

Die Position des mobilen Endgeräts (10) wird auf Basis von Aufnahmen einer Umgebung und Messen einer Beschleunigung des mobilen Endgeräts (10) bestimmt. Aus der Position des mobilen Endgeräts (10) am Nahpunkt und einer Position der Augen (31A, 32B) der Person kann dann die Nahpunktdistanz (31) bestimmt werden. Entsprechende Computerprogramme und ein entsprechende mobile Endgeräte werden ebenfalls bereitgestellt.

Fig. 3A

EP 3 944 806 A1

**Beschreibung**

Die vorliegende Patentanmeldung betrifft Verfahren und Vorrichtungen, insbesondere mobile Endgeräte, zum Bestimmen des Nahpunktes und der Nahpunktdistanz einer Person. Des Weiteren betrifft die Anmeldung Verfahren und Vorrichtungen zum Bestimmen einer sphärischen Brechkraft, insbesondere für eine Lesebrille oder einen Nahteil einer Gleitsichtbrille, unter Verwendung der so bestimmten Nahpunktdistanz und ein Verfahren zur Herstellung eines entsprechenden Brillenglases mit der so bestimmen Brillenstärke.

Unter der Akkommodationsbreite einer Person versteht man die Länge des visuellen Bereichs an Entfernungen vom Auge, in dem ein Auge der Person scharf sehen kann. Mit zunehmendem Alter wird die Akkommodationsbreite geringer, weil das Auge an Akkommodationskraft verliert, d.h. die Linse des Auges nur noch über einen zunehmend kleineren Bereich scharf stellen kann. Die Akkommodationsbreite ist durch die Entfernungsdifferenz zwischen dem Nahpunkt und dem Fernpunkt gegeben. Der Nahpunkt eines Auges ist dabei der dem Auge am nächsten gelegene Punkt, auf den das Auge eben noch scharf stellen kann. Dementsprechend ist der Fernpunkt der am weitesten entfernte Punkt, auf den das Auge noch scharf stellen kann. Die Begriffe Nahpunkt, Fernpunkt und Akkommodationsbreite sind in der DIN 5340 1998-04 definiert. Diese Norm definiert zudem den Nahpunktabstand eines Auges als Abstand des Nahpunktes vom objektseitigen Hauptpunkt des Auges definiert. Mit Nahpunktdistanz wird im Rahmen der vorliegenden Anmeldung allgemein der Abstand des Nahpunkts zu den Augen bezeichnet. Diese Nahpunktdistanz stellt einen Näherungswert für den normgemäßen Nahpunktabstand dar. Grundsätzlich kann der Nahpunkt für beide Augen einer Person unterschiedlich sein. Im Rahmen der vorliegenden Anmeldung wird in manchen Varianten auch ein näherungsweiser Wert für beide Augen als Nahpunktdistanz verwendet, beispielsweise ein Abstand des Nahpunkts zu einem Mittelpunkt zwischen der Pupillen der Augen bei Geradeausblick.

Während bei Kindern der Nahpunkt etwa 7 cm vom Auge entfernt liegt, also die Nahpunktdistanz etwa 7 cm beträgt, entfernt sich der Nahpunkt mit zunehmendem Alter immer weiter von den Augen, sodass die Nahpunktdistanz ansteigt und in höherem Alter Werte über 40 cm erreichen kann, sodass nähere Objekte nicht mehr scharf gesehen werden können. Dieses Phänomen wird als Altersweitsichtigkeit (Presbyopie) bezeichnet und ist unabhängig von sonstigen Fehlsichtigkeiten der Augen. Diese Altersweitsichtigkeit wird üblicherweise mit Lese- oder Nahbrillen korrigiert. Liegen gleichzeitig andere Fehlsichtigkeiten vor, können auch Gleitsichtbrillen mit einem Fernteil zum Sehen in die Ferne und einem Nahteil zur Korrektur dieser Altersweitsichtigkeit verwendet werden.

Zur Ermittlung der für die Fertigung von Brillengläsern derartiger Brillen benötigten sphärischen Brechkraft gemäß DIN EN ISO 13666: 2013 11.2 (hier für eine Lese- oder Nahbrille oder für den Nahteil einer Gleitsichtbrille), die üblicherweise in Dioptrien angegeben wird(siehe DIN EN ISO 13666: 2013 9.1), wird üblicherweise eine Untersuchung bei einem Optiker oder Augenarzt durchgeführt.

Diese Altersweitsichtigkeit bedingt durch die mit wachsendem Alter nachlassende Akkommodationsfähigkeit folgt typischerweise einem bekannten Verlauf, wie in H. Hofstetter, "Useful age-amplitude formula", Optom World, 38(12): 42-45, 1950, oder A. Duane, Normal values of the accommodation at all ages, J. Am. Med. Assoc. 59, 1010-1013, 1912, beschrieben. Stärkere Abweichungen von diesem Verlauf können auch Rückschlüsse auf ernstere gesundheitliche Veränderungen zulassen, wie zum Beispiel Diabetes (siehe C. I. Braun, W. E. Benson, N. A. Remaley, E. Y. Chew, und F. L. Ferris, "Accommodative amplitudes in the Early Treatment Diabetic Retinopathy Study", Retina, 15(4): 275-81, 1995 oder Nebenwirkungen von Medikamenten (siehe B. Gilmartin, "The Marton Lecture: Ocular Manifestations Of Systemic Medication", Ophthalmic and Physiological Optics, 7(4): 449-459, 1987).

Zur Messung der Akkommodationsbreite und insbesondere der Nahpunktdistanz sind unterschiedliche objektive und subjektive Messverfahren bekannt. Bei einem objektiven Messverfahren erfolgt dabei die Bestimmung des Akkommodationsbereichs ohne Rückmeldung der zu untersuchenden Person, während bei subjektiven Messverfahren eine Rückmeldung einer Person erfolgt, beispielsweise eine Rückmeldung, ob sie ein bestimmtes Objekt scharf sieht. Die vorliegende Anmeldung betrifft subjektive Messverfahren.

Klassische klinische Verfahren für die subjektive Bestimmung der Akkommodationsbreite, insbesondere der Nahpunktdistanz, sind sogenannte "Minus-to-blur"-Verfahren, wie sie in C. Sheard, "Dynamic Skiametry and Methods of Testing the Accommodation and Convergence of the Eyes", In Physiological optics, Seite 108, Cleveland Press, Chicago, 1920, beschrieben sind, und sogenannte "Push-up-pull-down"-Verfahren, wie sie in F. Donders and W. Moore, "On the anomalies of accommodation and refraction of the eye with a preliminary essay on physiological dioptrics", The New Sydenham Society, London, 1864, beschrieben sind.

Bei letzterem Verfahren wird ein sogenannter R.A.F.-Maßstab ("Royal Air Force Rule") auf dem Gesicht einer zu untersuchenden Person aufgesetzt. Auf diesem Maßstab ist eine Sehtafel mit Sehzeichen, beispielsweise einer Schrift, montiert. Eine behandelnde Person (zum Beispiel Arzt oder Optiker) bewegt diese Sehtafel nun langsam auf den Patienten zu bzw. von ihm weg, bis dieser meldet, dass er die Sehzeichen auf der Sehtafel nicht mehr bzw. wieder scharf sehen kann. Die Distanz der Sehtafel zum Patienten, genauer gesagt zum Auge des Patienten oder zu den Augen des Patienten, wird dann auf dem R.A.F.-Maßstab abgelesen und ergibt die Nahpunktdistanz. Diese Untersuchung kann sowohl monokular, d.h. mit einem Auge, als auch binokular, d.h. mit beiden Augen, durchgeführt werden. Eine Erläuterung dieser Messmethode ist auch in dem Video abrufbar unter https://www.youtube.com/watch?v=JjrRtH0qk_0 zu sehen.

Dieses klassische "Push-up-pull-down"-Verfahren hat einige Nachteile. Insbesondere muss ein relativer unbequemer Maßstab im Gesicht des Patienten gehalten werden, das Verfahren ist nicht alleine durchführbar, sondern benötigt eine untersuchende Person, und der Messstab ist ein recht sperriges Gerät. Daher existieren Bemühungen, dieses klassische Verfahren zu verbessern und/oder zu automatisieren.

So wird in Ide, Takeshi et al., "The Effect of 3D Visual Simulator on Children's Visual Acuity - A Pilot Study Comparing Two Different Modalities", The Open Ophthalmology Journal 7 (2013): 69-48, PMC, Web, 11. April 2018, eine Vorrichtung vorgestellt, bei welcher ein Fixationstarget motorisiert auf die zu untersuchende Person zubewegt wird, um den Akkommodationsbereich der Person zu bestimmen. Ein Fixationstarget ist dabei eine Komponente, auf die die zu untersuchende Person ihren Blick richten soll. Ein derartiges Fixationstarget kann insbesondere die oben erwähnten Sehzeichen beinhalten. Diese Vorrichtung erlaubt durch die motorisierte Bewegung des Fixationstargets bereits eine gewisse Automatisierung, ist jedoch nach wie vor unhandlich und wird typischerweise bei Optikern oder Ärzten eingesetzt.

In Ide, Takeshi & Negishi, Kazuno & Yamaguchi, Takefumi & Hara, Shuya & Toda, Ikuko & Tsubota, Kazuo, (2012), "New Compact Accommodometer to Measure Accommodation Amplitude as a Biomarker", The Asia-Pacific Journal of Ophthalmology, 1. 24-27, 1097/APO.0b013e31823f1a69, wird eine Vorrichtung beschrieben, welche eine Papiersehtafel, einen Ultraschalldistanzsensor und eine entsprechende Ausleseelektronik aufweist. Dieses Gerät kann in der Hand einer Person gehalten werden und erlaubt eine Bestimmung der Nahpunktdistanz der Person durch diese selbst, ohne dass die Anwesenheit eines Arztes oder Optikers nötig ist. Allerdings ist hier nach wie vor ein spezielles Gerät erforderlich.

In Alec Kingsnorth, "Technological enhancements to optometric clinical tests", Dissertation, Aston University, März 2015, wird ein Gerät zur Bestimmung der Nahpunktdistanz beschrieben, welches ein Smartphone als Anzeigeeinheit für Sehzeichen verwendet. Eine Abstandsmessung zu einem Kopf der zu untersuchenden Person wird mittels Ultraschallsensoren durchgeführt. Die hierzu nötigen Ultraschallsensoren und eine entsprechende Mikrosteuerung sind in einer Hülle für das Smartphone untergebracht. Hier wird also eine zusätzliche Komponente zum Smartphone benötigt.

Die US 2016/0120402 A1 offenbart Verfahren zum Ermitteln einer Refraktion des Auges mithilfe eines Smartphones, bei welchem der Fernpunkt des Auges bestimmt wird. Dabei wird die Distanz zwischen Auge und dem Smartphone mittels einer Kamera und einem Vergleich mit einem Objekt bekannter Größe, wie zum Beispiel einer Kreditkarte, bestimmt. Es ist zudem die Möglichkeit erwähnt, einen Beschleunigungssensor zur Distanzbestimmung zu verwenden. Hierfür ist ein kalibrierter Anfangsabstand nötig, wie beispielsweise ein Abstand von Null direkt am Auge oder eine andere festgelegte Abstandsdistanz vom Auge, um dann durch Integration der Beschleunigung die Distanz zu bestimmen. Hier wird also entweder ein zusätzliches Hilfsmittel, nämlich der Gegenstand bekannter Abmessung, oder ein fester Referenzpunkt benötigt.

Aus der WO 2018/002332 A2 sind Verfahren zur subjektiven Refraktionsbestimmung mittels exzentrischer Photorefraktion bekannt, bei welchen ebenfalls verschiedene Möglichkeiten zur Abstandsmessung zwischen einem Smartphone und einem Kopf einer Person zum Einsatz kommen. Diese benötigen entweder Hilfsmittel, wie den oben erwähnten Gegenstand bekannter Abmessungen, Eingaben eines Benutzers oder sie benutzen einen Autofokus einer Kamera des Smartphones, welcher je nach Lichtverhältnissen ungenau sein kann.

Es ist daher, ausgehend beispielsweise von der US 2016/0120402 A1, eine Aufgabe, Möglichkeiten zur Bestimmung des Nahpunkts und weitergehend der Nahpunktdistanz mittels mobilen Endgeräten, wie Smartphones, bereitzustellen, bei welchen keine zusätzlichen Hilfsmittel, wie Gegenstände bekannter Größe, und auch kein vorgegebener Startpunkt nötig ist und eine ausreichend hohe Genauigkeit bei der Distanzbestimmung erreicht werden kann. Weitergehend hierzu sollen Möglichkeiten zur Bestimmung einer sphärischen Brechkraft für ein Brillenglas einer Lese- oder Nahbrille oder für den Nahteil einer Gleitsichtbrille unter Verwendung der so bestimmten Nahpunktdistanz sowie zur Fertigung von Brillengläsern bereitgestellt werden.

Diese Aufgabe wird gelöst durch ein Verfahren zur Bestimmung des Nahpunkts gemäß Anspruch 1 und ein mobiles Endgerät gemäß Anspruch 15. Die abhängigen Ansprüche definieren weitere Ausführungsbeispiele, Verfahren zur Bestimmung der Nahpunktdistanz, zur Bestimmung eines sphärischen Brechwertes und zum Herstellen eines Brillenglases sowie ein entsprechendes Computerprogramm zur Durchführung des Verfahrens auf einem mobilen Endgerät.

Erfindungsgemäß wird ein computerimplementiertes Verfahren zum Bestimmen des Nahpunkts einer Person mit einem mobilen Endgerät bereitgestellt, umfassend:

Bestimmen der Position des mobilen Endgeräts an dem Nahpunkt.

Das Verfahren ist dadurch gekennzeichnet, dass das Bestimmen der Position des mobilen Endgeräts an dem Nahpunkt auf Basis wiederholter Aufnahmen einer Umgebung des mobilen Endgeräts mit einer eingebauten Kamera des mobilen Endgeräts und einem wiederholten Messen einer Beschleunigung des mobilen Endgeräts mit einem Beschleunigungssensor des mobilen Endgeräts erfolgt.

Auf diese Weise kann das Bestimmen der Position des mobilen Endgeräts an dem Nahpunkt, welche dann zumindest als Näherungswert für die Position des Nahpunkts verwendet werden kann, mittels eingebauter Sensoren des mobilen Endgeräts, nämlich einer Kamera und einem Beschleunigungssensor, erfolgen, welche in handelsüblichen mobilen Endgeräten, wie Smartphones oder Tablet-PCs, ohnehin vorhanden sind. Dabei wird keine zusätzliche Ausrüstung für das mobile Endgerät, wie Ultraschallsensoren oder ein Gegenstand bekannter Größe, und auch keine definierte Startposition benötigt.

Unter einem mobilen Endgerät ist eine Vorrichtung zu verstehen, welche zumindest einen programmierbaren Prozessor sowie eine Kamera und einen Beschleunigungssensor umfasst, und welche dazu ausgelegt ist, getragen zu werden, d.h. hinsichtlich Dimensionen und Gewicht derart ausgestaltet ist, dass sie von einer Person mitführbar ist. Es können weitere Komponenten vorhanden sein, wie zum Beispiel eine Anzeige. Typische Beispiele für derartige mobile Endgeräte sind Smartphones oder Tablet-PCs, die heutzutage über einen Sensorbildschirm (Touchscreen), eine oder mehrere Kameras, Beschleunigungssensoren und anderen Komponenten, wie drahtlosten Schnittstellen für Mobilfunk oder WLAN (Wireless LAN) aufweisen. Das Gewicht derartiger mobiler Endgeräte liegt typischerweise unter 2 kg, meist unter 1 kg und darunter.

Unter einer Position ist eine Position in einem ortsfesten Koordinatensystem zu verstehen, d.h. in einem Koordinatensystem, welches sich bei der Bewegung des mobilen Endgeräts nicht mitbewegt. Ein derartiges Koordinatensystem wird auch als Weltkoordinatensystem bezeichnet. Eine Angabe ist beispielsweise in kartesischen Koordinaten oder in Kugelkoordinaten möglich. Da bei manchen Anwendungen letztendlich eine Distanz, d.h. ein Unterschied zwischen zwei Positionen, bestimmt werden soll, kann der Ursprung des Koordinatensystems, in dem die Position bestimmt wird, in vielen Fällen frei gewählt werden. Beispielsweise kann die Position des mobilen Endgeräts zum Zeitpunkt des Beginn des Verfahrens als Koordinatenursprung festgelegt werden, es ist jedoch auch jede beliebige andere Wahl des Koordinatenursprungs möglich.

Durch das wiederholte Aufnehmen der Umgebung und das wiederholte Messen der Beschleunigung ist eine absolute Positionsbestimmung während der Bewegung möglich. Eine solche Vorgehensweise wird auch als visuell-inertiale Odometrie bezeichnet und ist beispielsweise in G. Nützli et al., "Fusion of IMU and Vision for absolute scale estimation in monocular SLAM", J. Intel. Robot Syst. 61, 2011, Seiten 287-299, detailliert erläutert und ermöglicht neben der Bestimmung der Position in einem Weltkoordinatensystem auch die Bestimmung der Orientierung des mobilen Endgeräts, welche als Verkippung eines mit dem mobilen Endgerät verbundenen Koordinatensystems gegenüber dem Weltkoordinatensystem beschrieben werden kann. Die Orientierung wird, wie weiter unten erläutert, bei manchen Ausführungsformen zur Bestimmung der Position der Augen verwendet. Die Kombination aus Position und Orientierung wird auch als Pose bezeichnet, siehe beispielsweise DIN EN ISO 8373: 2012.

An die Umgebung werden dabei keine besonderen Anforderungen gestellt, es müssen lediglich identifizierbare Punkte, beispielsweise Kanten von Objekten, und dergleichen, vorhanden sein. Diese sind in einer üblichen Umgebung, beispielsweise einem möblierten Zimmer, stets vorhanden.

Das Verfahren kann weiter ein Anzeigen von Sehzeichen auf dem mobilen Endgerät umfassen. Anhand dieser Sehzeichen kann die Person beurteilen, ob der Nahpunkt erreicht ist, beispielsweise wenn die Sehzeichen bei einer Bewegung des mobilen Endgeräts auf die Person zu gerade noch scharf erscheinen. Eine Anzeige ist auf mobilen Endgeräten wie Smartphones oder Tablet-PCs ohnehin vorhanden, so dass vorhandene Hardware genutzt werden kann.

Unter Sehzeichen sind Zeichen zu verstehen, welche die Person betrachten kann. Verschiedene Arten von Sehzeichen können verwendet werden, beispielsweise standardisierte Optotypen (z.B. Landoltringe, Snellen-Haken, ggf. mit daran angeschlossenen weiteren Sehzeichen, wie zum Beispiel Buchstaben oder Zahlen), Bilder, Sinusgitter mit unterschiedlichen Ortsfrequenzen bei gleichen oder variierendem Kontrast, Zahlen, Buchstaben oder Symbole. Typischerweise werden dabei Sehzeichen in verschiedenen Größen dargestellt. Wie bereits eingangs erläutert, ist der Nahpunkt dann der am nächsten an den Augen oder einem Auge liegende Punkt, an dem die Sehzeichen scharf gesehen werden können.

Das Verfahren kann weiter umfassen:

Auffordern der Person, das mobile Endgerät zu dem Nahpunkt der Person zu bewegen, und
Erhalten einer Rückmeldung von der Person, wenn das mobile Endgerät an dem Nahpunkt der Person ist.

Das Auffordern der Person kann dabei durch eine Ausgabe des mobilen Endgeräts geschehen. Beispielsweise kann die Aufforderung schriftlich oder mittels Symbolen auf der Anzeige des mobilen Endgeräts erfolgen, oder durch eine Sprachausgabe mittels eines Lautsprechers des mobilen Endgeräts. Die Person wird dabei typischerweise dazu aufgefordert, das mobile Endgerät mit den darauf angezeigten Sehzeichen von einer von den Augen möglichst weit entfernten Position (beispielsweise eine Position mit ausgestrecktem Arm der Person) auf das Gesicht zuzubewegen, bis der Nahpunkt erreicht ist. Grundsätzlich ist jedoch z.B. auch eine Bewegung von einer Position direkt bei den Augen vom Gesicht weg möglich. Auf diese Weise ist keine Anleitung durch eine entsprechend geschulte weitere Person wie einen Optiker erforderlich, sondern das mobile Endgerät kann der Person direkt die Anweisungen zur Durchführung des Verfahrens geben.

Das Erhalten der Rückmeldung kann über irgendeine Eingabemodalität des mobilen Endgeräts erfolgen. Beispielsweise kann ein Taster an dem mobilen Endgerät betätigt werden, eine bestimmte Stelle auf einem Sensorbildschirm (Touchscreen), der auch zum Anzeigen der Sehzeichen dient, des mobilen Endgeräts kann berührt werden, oder es kann eine Rückmeldung durch die Stimme der Person (beispielsweise durch Aussprache eines bestimmten Worts wie "jetzt" oder dergleichen) erreicht werden, welche über ein eingebautes Mikrofon des mobilen Endgeräts empfangen wird. Derartige eingebaute Mikrofone sind bei mobilen Endgeräten wie Smartphones oder Tablet-PCs typischerweise vorhanden.

Die eingebaute Kamera kann dabei insbesondere eine Kamera sein, welche auf einer Anzeige des mobilen Endgeräts, die zum Anzeigen von Sehzeichen verwendet wird, gegenüberliegenden Seite angeordnet ist. Diese Seite wird bei Smartphones oder Tablet-PCs auch als Rückseite bezeichnet, und die entsprechende Kamera wird als Rückkamera (Englisch: back camera) bezeichnet. Wenn bei der Durchführung des Verfahrens die Anzeige der untersuchten Person zugewandt ist (z.B. weil die Person ja die Schärfe der angezeigten Sehzeichen beurteilen soll), ist somit die Rückkamera der Person abgewandt und kann die Umgebung aufnehmen, ohne dass die Person den "Blick" der Kamera auf die Umgebung verstellt.

Weitergehend wird ein computerimplementiertes Verfahren zum Bestimmen des Nahpunkts einer Person mit einem mobilen Endgerät bereitgestellt, bei dem die Position des Nahpunkts wie oben erläutert bestimmt wird. Weiter umfasst das Verfahren:

Bestimmen der Position von mindestens einem Auge der Person, und
Bestimmen der Nahpunktdistanz auf Basis der Position des mobilen Endgeräts an dem Nahpunkt und der Position des mindestens einen Auges.

So kann die Nahpunktdistanz mit der vorhandenen Hardware von mobilen Endgeräten wie Smartphones oder Tablet-PCs bestimmt werden.

Die Position des mindestens einen Auges gibt dabei an, an welchem Ort sich ein Auge oder beide Augen der Person befinden. Sie wird im Folgenden auch einfach als Position der Augen der Person bezeichnet, wobei zu verstehen ist, dass es sich auch um eine Position von nur einem Auge handeln kann. Wie später erläutert werden wird, kann dies auch eine näherungsweise Position sein. Die Position kann für beide Augen der Person getrennt bestimmt werden. Als Position jedes Auges kann dabei beispielsweise die Position der Pupillen oder ein Mittelpunkt zwischen innerem und äußerem Augenwinkel verwendet werden. Bei anderen Ausführungsformen, die später noch näher erläutert werden, kann die Position beider Augen auch als näherungsweise Position auf Basis einer Position des mobilen Endgeräts, welches direkt an den Kopf gehalten wird, bestimmt werden.

Die Nahpunktdistanz kann dann als Differenz zwischen der Position des mobilen Endgeräts an dem Nahpunkt und einem Punkt, welcher die Position des mindestens einen Auges repräsentiert, bestimmt werden, und ist wie oben erläutert ein Näherungswert für den Nahpunktabstand nach DIN 5340 1998-04. Wenn die Positionen beider Augen getrennt bestimmt werden, kann dieser Punkt, welcher dann die Position beider Augen repräsentiert, ein Mittelpunkt einer Strecke, die die Positionen der beiden Augen verbindet, sein. Grundsätzlich ist jedoch auch eine Bestimmung der Nahpunktdistanz für beide Augen getrennt oder nur für ein Auge möglich, indem immer ein Auge abgedeckt wird und der Nahpunkt für das jeweils nicht abgedeckte Auge bestimmt wird. In diesem Fall ist die Nahpunktdistanz für das jeweilige Auge eine Differenz zwischen der Position des Auges und der Position des mobilen Endgeräts.

Bei Verfahren, bei denen die Position der Augen als Position der Pupillen der Augen bestimmt wird, kann optional zusätzlich der Pupillenabstand gemäß DIN 33402-2, Tabelle 69, als Abstand der Pupillen berechnet werden. Dieser Pupillenabstand (abgekürzt PD) wird bei der Brillenanpassung benötigt. Sie kann in diesem Fall ohne größeren Aufwand mitbestimmt werden, sodass keine spätere Bestimmung mehr gebraucht wird.

Bei einer ersten Variante zur Bestimmung der Position der Augen wird während der Bewegung des mobilen Endgeräts zum Nahpunkt nicht nur die Position des mobilen Endgeräts, sondern auch die Orientierung des mobilen Endgeräts, also insgesamt die Pose des mobilen Endgeräts, mittels visuell-inertialer Odometrie bestimmt. Zudem wird während der Bewegung wiederholt eine Bild der Augen der Person aufgenommen. Die Position der Augen wird dann auf Basis der Posen des mobilen Endgeräts während der Bewegung und der Bilder der Augen während der Bewegung bestimmt.

Hierzu werden mittels Bildverarbeitung die Augen, beispielsweise die Pupillen hiervon, in den Bildern identifiziert und die Position der Augen wird dann ähnlich einer Triangulation bestimmt. Ein geeignetes Bildverarbeitungsverfahren zur Bestimmung der Position der Augen ist beispielsweise in F. Timm und E. Barth, "Accurate eye centre localisation by means of gradients", Proceedings of the Int. Conference on Computer Theory and Applications (VISAPP), Vol. 1, Seiten 125-130, Algarve, Portugal, 2011, beschrieben. Für eine derartige Bestimmung der Position der Augen werden die Posen des mobilen Endgeräts sowie die entsprechenden Bilder der Augen an mindestens zwei Punkten der Bewegung des mobilen Endgeräts benötigt. Die Triangulation ist, wie in dem Wikipedia-Artikel "Triangulation (Messtechnik)" erläutert, dabei eine geometrische Methode zur optischen Abstandsmessung (Stand 4. Februar 2019). Auf Basis davon, unter welchen Winkeln die Augen an den jeweiligen Posen in dem Bild gesehen werden, kann dann die Position der Augen bestimmt werden. Diese Herangehensweise zur Bestimmung der Position der Augen wird später bei der Figurenbeschreibung noch detailliert erläutert.

Auf diese Weise kann die Position der Augen während der Bewegung des mobilen Endgeräts zum Nahpunkt bestimmt werden, sodass zur Bestimmung der Position der Augen nicht noch einmal eine separate Messung durchgeführt werden muss.

Die Bilder der Augen der Person können dabei insbesondere mit einer sogenannten Frontkamera, d.h. einer Kamera, die auf einer gleichen Seite des mobilen Endgeräts angeordnet ist, wie die Anzeige, auf der die Sehzeichen angezeigt werden, aufgenommen werden. Wenn die Person bei bestimmungsgemäßem Gebrauch beispielsweise auf die Sehzeichen oder auch sonst auf die Anzeige des mobilen Endgeräts blickt, sind die Augen dann im Blickfeld einer derartigen Frontkamera. Die meisten heutzutage erhältlichen Smartphones und Tablets weisen eine derartige Frontkamera auf, sodass hierfür keine spezielle Hardware benötigt wird.

Auch wenn grundsätzlich zur Bestimmung der Position der Augen Bilder der Augen an zwei Posen des mobilen Endgeräts ausreichend sind, erfolgt das Aufnehmen der Bilder der Augen bevorzugt an mehr als zwei Posen, um dann durch Mittelung die Messgenauigkeit erhöhen zu können.

Bei einer alternativen Herangehensweise umfasst das Bestimmen der Position der Augen der Person folgende Schritte:

Bestimmen der Position des mobilen Endgeräts an den Augen auf Basis wiederholter Aufnahmen der Umgebung des mobilen Endgeräts mit der eingebauten Kamera des mobilen Endgeräts und wiederholtem Messen der Beschleunigung des mobilen Endgeräts mit dem eingebauten Beschleunigungssensor während der Bewegung des mobilen Endgeräts zu den Augen mittels visuell-inertialer Odometrie, und
Bestimmen der Position der Augen basierend auf der Position des mobilen Endgeräts an den Augen.

Hier wird also die Position der Augen im Wesentlichen genauso wie oben für die Position des Nahpunkts erläutert mittels visuell-inertialer Odometrie bestimmt.

Dabei kann das Verfahren ein Auffordern der Person, das mobile Endgerät zu den Augen der Person zu bewegen, und ein Erhalten einer Rückmeldung, wenn das mobile Endgerät an den Augen der Person ist, umfassen, um der Person ähnlich wie bei der Bestimmung der Position des Nahpunkts Anweisungen zu geben.

Die Position des mobilen Endgeräts bei den Augen kann dabei direkt als die Position der Augen verwendet werden. Es kann jedoch auf die Position des Endgeräts auch noch ein bestimmter Versatz, beispielsweise 1 cm oder 0,5 cm, in Richtung der Augen hinzugerechnet werden, um zu berücksichtigen, dass beispielsweise aufgrund der Nase der Person immer ein gewisser Restabstand des mobilen Endgeräts zu den Augen verbleiben wird. Dieser Versatz kann ebenso bei der Bestimmung der Nahpunktdistanz berücksichtigt werden. In diesem Fall wird die Position der Augen ohnehin als ein gewisser Mittelwert, nämlich als die Position des mobilen Endgeräts oder auf Basis desselben, bestimmt, und nicht zwischen den beiden Augen unterschieden.

Bei dieser Art der Bestimmung ist keine Frontkamera nötig. Wichtig ist dabei jedoch, dass die Person die Position des Kopfs zwischen der Bestimmung der Position des Nahpunkts und der Bestimmung der Position der Augen möglichst nicht verändert.

Das Bestimmen der Nahpunktdistanz wie oben erläutert kann mehrmals wiederholt werden, um durch Mittelung über mehrere Messungen die Genauigkeit zu verbessern. Dabei kann die Person auch angewiesen werden, das mobile Endgerät einmal von weiter weg auf die Augen zuzubewegen, bis der Nahpunkt erreicht ist, und einmal von einer Position unmittelbar bei den Augen von den Augen wegzubewegen, bis der Nahpunkt erreicht ist.

Wenn das Verfahren von der Person öfter in größeren zeitlichen Abständen durchgeführt wird, kann auch überprüft werden, ob die Nahpunktdistanz einem erwarteten Verlauf folgt, um gegebenenfalls Krankheiten erkennen zu können, wie eingangs erläutert.

Weitergehend wird ein Verfahren zum Bestimmen eines sphärischen Brechwertes für ein Brillenglas bereitgestellt, bei dem die Nahpunktdistanz einer Person nach einem der oben erläuterten Verfahren bestimmt wird. Die Brillenstärke wird dann auf Basis der Nahpunktdistanz und einer Lesedistanz bestimmt.

Unter der sphärischen Brechkraft (kurz als Sphäre bezeichnet) gemäß DIN EN ISO 13666: 2013.11.2 für eine Nahbrille oder Lesebrille oder eine entsprechende sphärische Brechkraft für einen Nahteil einer Gleitsichtbrille zu verstehen.

Unter der Lesedistanz versteht man einen Abstand von den Augen, in dem ein zu lesendes Objekt von der Person üblicherweise gehalten wird. Der Begriff "Lesen" ist dabei breit zu verstehen und kann auch ein Betrachten von Bildern und dergleichen beinhalten. Ein zu lesendes Objekt kann beispielsweise ein Buch sein, kann aber auch ein mobiles Endgerät wie ein Smartphone oder ein Tablet-PC sein, auf dem heutzutage häufig Texte gelesen werden (beispielsweise E-Mails oder Texte auf Internetseiten).

Bei einer Variante wird die Lesedistanz fest vorgegeben, beispielsweise auf einen Bereich zwischen 20 cm und 40 cm, beispielsweise auf 30 cm festgesetzt. Dies hat den Vorteil, dass keine speziellen Maßnahmen zum Bestimmen der Lesedistanz ergriffen werden müssen, bringt aber eine gewisse Ungenauigkeit mit sich, da diese Lesedistanz für alle Personen angenommen wird und nicht individuell bestimmt wird.

Bei einer weiteren Ausführungsform wird der Benutzer aufgefordert, die Lesedistanz einzugeben, und die so eingegebene Lesedistanz wird verwendet. Hier ist eine größere Individualisierung möglich, allerdings muss in diesem Fall die Person ihre Lesedistanz selbst abschätzen.

Bei einer weiteren Ausführungsbeispiel umfasst das Verfahren ein Bestimmen der Lesedistanz mit folgenden Schritten:

Bestimmen der Position des mobilen Endgeräts in der Leseposition auf Basis von wiederholtem Aufnehmen der Umgebung des mobilen Endgeräts mit der eingebauten Kamera des mobilen Endgeräts und wiederholtem Messen der Beschleunigung des mobilen Endgeräts mit dem eingebauten Beschleunigungssensor während einer Bewegung des mobilen Endgeräts mittels in die Leseposition, und

Bestimmen der Lesedistanz auf Basis der Position des mobilen Endgeräts in der Leseposition und der Position der Augen.

Hierzu können der Person wieder Anweisungen gegeben werden. Hierzu kann das Verfahren umfassen:

Auffordern der Person, das mobile Endgerät in eine Leseposition zu bringen,
Erhalten einer Rückmeldung, wenn das mobile Endgerät in der Leseposition ist.

Dabei kann die Position der Augen auch wie oben erläutert mittels Aufnahmen von Bildern der Augen während der Bewegung neu bestimmt oder aktualisiert werden, um Fehler durch eine Veränderung der Position der Augen zu verringern.

Die Lesedistanz ergibt sich dann als Differenz zwischen der Position der Augen und der Position des mobilen Endgeräts in der Lesedistanz, wobei für die Position der Augen die obigen Ausführungen gelten.

Auf diese Weise kann die Lesedistanz also individuell bestimmt werden. Die Verwendung des mobilen Endgeräts hierfür hat auch den Vorteil, dass speziell eine Lesedistanz für mobile Endgeräte bestimmt wird, die heutzutage häufiger betrachtet werden als herkömmliche Leseobjekte wie Bücher.

Das Verfahren wird dabei vom Benutzer mit korrekt korrigierten Sehfehlern durchgeführt, d.h. einer Person, die entweder abgesehen von der oben erläuterten Altersweitsichtigkeit keine Sehfehler hat oder bei der sonstige Sehfehler bereits - beispielsweise durch eine geeignete Brille - korrigiert sind. Der sphärische Brechwert ergibt sich dann gemäß $R_{read} = (1/a_{read}) - (1/a_p) + R_{korr}$. Dabei ist $R_{read}$ der benötigte sphärische Brechwert der Lesebrille (in Dioptrien), $a_{read}$ die Lesedistanz, $a_p$ die Nahpunktdistanz und $R_{korr}$ ein Brechwert (in Dioptrien) zur Korrektur übriger Sehfehler, d.h. der Brechwert eines Brillenglases, die die Person ohne die oben erwähnte Altersweitsichtigkeit benötigen würde. Für Benutzer ohne sonstige Sehfehler, d.h. Personen, die sonst keine Brille tragen, gilt $R_{korr} = 0$. Der Kehrwert der Nahpunktdistanz $1/a_p$ wird auch als Nahpunktrefraktion $A_P$ bezeichnet.

Des Weiteren wird auch ein Verfahren zum Herstellen eines Brillenglases bereitgestellt, bei welchem der sphärische Brechwert wie oben erläutert ermittelt wird und dann ein entsprechendes Brillenglas hergestellt wird. Hierzu kann der ermittelte sphärische Brechwert von dem mobilen Endgerät an einen Brillenglashersteller übermittelt werden, beispielsweise durch in dem mobilen Endgerät vorhandene Schnittstellen zur drahtlosen Kommunikation.

Zudem wird ein Computerprogramm bereitgestellt, umfassend Befehle, die bei der Ausführung des Programms durch ein mobiles Endgerät dieses veranlasst, eines der oben beschriebenen Verfahren zum Bestimmen der Nahpunktdistanz oder der Brillenstärke durchzuführen. Derartige Computerprogramme für mobile Endgeräte werden häufig auch als "Apps" bezeichnet. Ein entsprechendes computerlesbares Speichermedium mit einem derartigen Computerprogramm und entsprechendes Datenträgersignal werden ebenfalls bereitgestellt.

Schließlich wird ein mobiles Endgerät bereitgestellt, umfassend:

eine Anzeige,
eine Kamera,
einen Beschleunigungssensor, und
einen Prozessor, der so konfiguriert ist, dass er bewirkt, dass eines der oben beschriebenen Verfahren zum Bestimmen des Nahpunkts, der Nahpunktdistanz oder des sphärischen Brechwertes auf dem mobilen Endgerät ausführt, also zumindest die folgenden Schritte durchgeführt werden:

Bestimmen der Position des mobilen Endgeräts an dem Nahpunkt,
wobei das Bestimmen der Position des mobilen Endgeräts an dem Nahpunkt auf Basis wiederholter Aufnahmen einer Umgebung des mobilen Endgeräts mit einer eingebauten Kamera des mobilen Endgeräts und auf Basis von wiederholtem Messen einer Beschleunigung des mobilen Endgeräts mit einem eingebauten Beschleunigungssensor des mobilen Endgeräts während einer Bewegung des mobilen Endgeräts zum Nahpunkt erfolgt.

Hierzu kann ein entsprechendes Computerprogramm in einem Speicher des mobilen Endgeräts bereitgestellt sein oder das mobile Endgerät kann eintsprechende Mittel umfassen, die die Verfahrensschritte durchführen.

Auf diese Weise kann mit einem mobilen Endgerät die Nahpunktdistanz bestimmt werden und in Weiterbildungen dann die Brillenstärke bestimmt werden.

Ausführungsbeispiele der Erfindung werden nachfolgend unter Bezugnahme auf die beigefügten Zeichnungen näher erläutert. Es zeigen:

Fig. 1 ein Blockdiagramm eines mobilen Endgeräts, wie es bei verschiedenen Ausführungsbeispielen Verwendung findet,

Fig. 2 ein Flussdiagram eines Verfahrens gemäß einem Ausführungsbeispiel,

Fig. 3A und 3B Diagramme zur Veranschaulichung verschiedener Verfahrensschritte der Fig. 2,

Fig. 4 ein Diagramm zur Veranschaulichung einer Bestimmung einer Position der Augen gemäß mancher Ausführungsbeispiele,

Fig. 5 ein Flussdiagram eines Verfahrens gemäß einem weiteren Ausführungsbeispiel, und

Fig. 6A bis 6C Diagramme zur Veranschaulichung mancher Verfahrensschritte der Fig. 5.

DETAILLIERTE BESCHREIBUNG

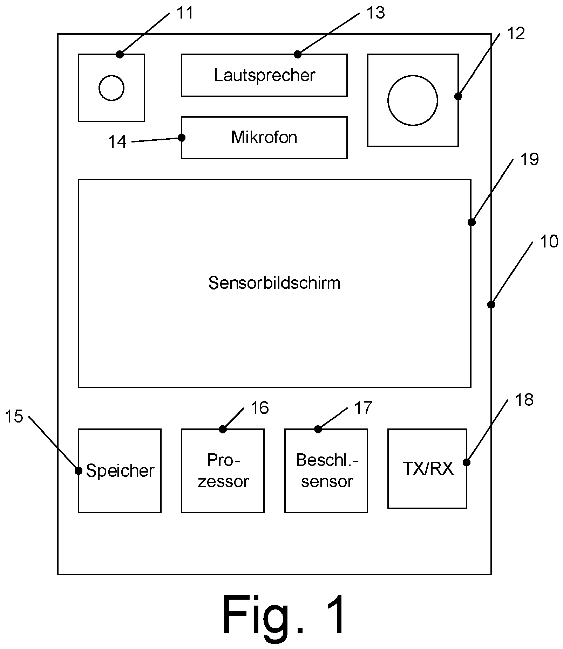

Die im Folgenden beschriebenen Ausführungsbeispiele verwenden ein mobiles Endgerät zur Bestimmung der Nahpunktdistanz einer Person und weitergehend zur Bestimmung eines sphärischen Brechwertes, auf deren Basis dann Brillengläser gefertigt werden können. Die Fig. 1 zeigt ein Blockdiagramm eines mobilen Endgeräts 10, wie es bei derartigen Ausführungsbeispielen verwendet werden kann. Das mobile Endgerät kann dabei ein Smartphone oder ein Tabletcomputer sein, welche typischerweise zumindest die in Fig. 1 dargestellten Komponenten enthalten.

Das mobile Endgerät 10 der Fig. 1 weist einen Sensorbildschirm 19 (Englisch: "touchscreen") auf, welcher als Eingabegerät sowie zur Ausgabe und bei der Durchführung erfindungsgemäßer Verfahren zum Anzeigen von Sehzeichen dient. Gesteuert wird das mobile Endgerät 10 von einem Prozessor 16, der auf einen Speicher 15 zugreifen kann, in dem Computerprogramme abgelegt sein können. Wie bereits erwähnt werden derartige Computerprogramme für mobile Endgeräte auch als Apps bezeichnet. Das mobile Endgerät 10 verfügt weiterhin über einen Lautsprecher 13 zur Ausgabe von Tönen und über ein Mikrofon 14, über das beispielsweise Sprachbefehle entgegengenommen werden können. Das mobile Endgeräte 10 weist weiter eine Frontkamera 11 und eine Rückkamera 12 auf. Die Frontkamera 11 ist dabei auf der gleichen Seite wie der Sensorbildschirm 19 angeordnet, sodass mittels der Kamera 11 eine Person, insbesondere die Augen einer Person, die den Sensorbildschirm 19 betrachtet, aufgenommen werden kann. Die Rückkamera 12 ist auf der dem Sensorbildschirm 19 gegenüberliegenden Seite des mobilen Endgeräts 10 angeordnet.

Des Weiteren weißt das mobile Endgerät 10 einen Beschleunigungssensor 17 auf, mit welchen Beschleunigungen des mobilen Endgeräts 10 gemessen werden können. Schließlich ist eine Kommunikationsschaltung 18 zum Senden (TX, vom Englischen: transmit) und Empfangen (RX, vom Englischen: receive) von Daten bereitgestellt, beispielsweise über ein Mobilfunknetz und/oder über ein WLAN-Netz (Wireless LAN).

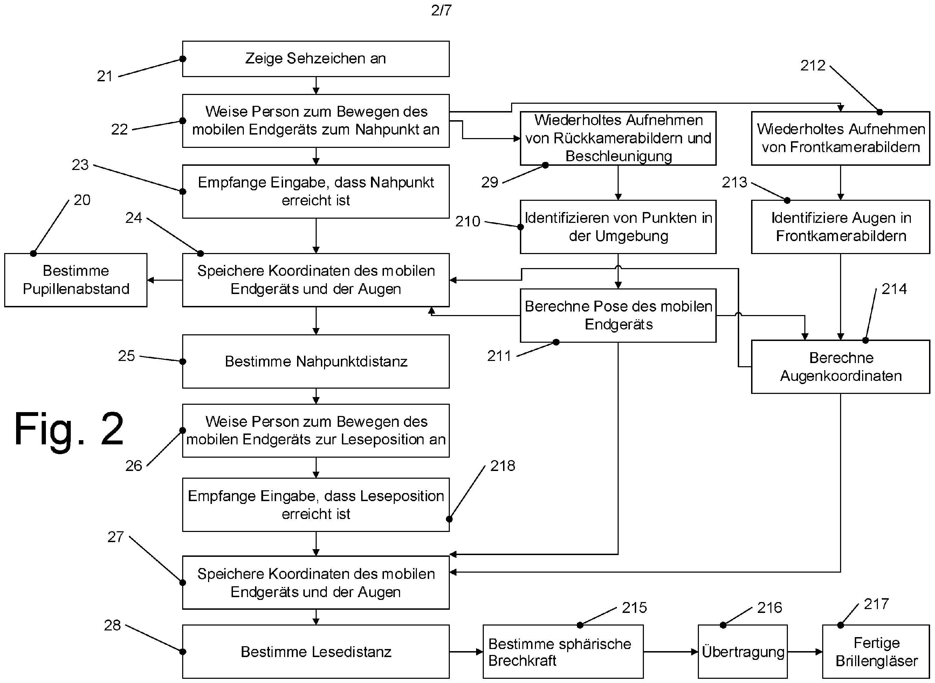

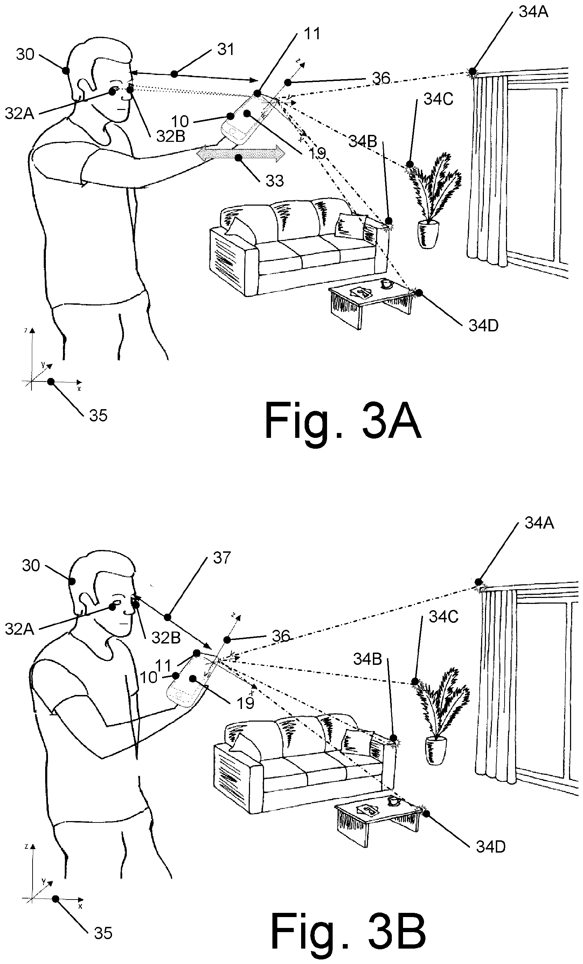

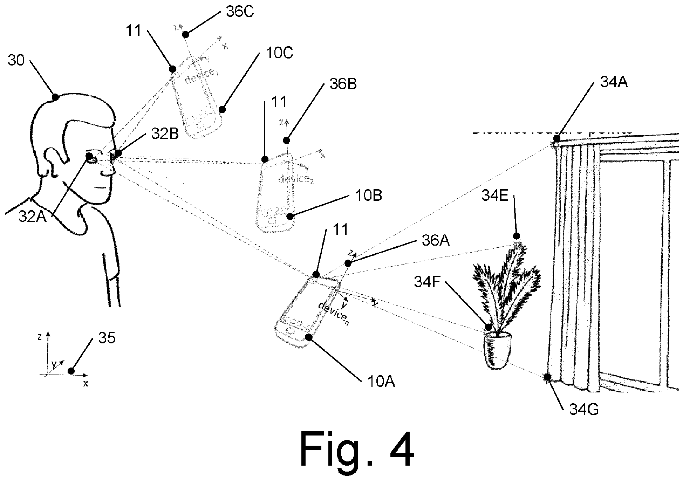

Die Fig. 2 zeigt ein Flussdiagram eines Verfahrens gemäß einem Ausführungsbeispiel. Das Verfahren der Fig. 2 kann mit dem mobilen Endgerät 10 der Fig. 1 ausgeführt werden und wird unter Bezugnahme auf das mobile Endgerät 10 der Fig. 1 beschrieben. Zur zusätzlichen Erläuterung des Verfahrens der Fig. 2 wird auf die Fig. 3A, 3B und 4 Bezug genommen.

In Schritt 21 werden Sehzeichen auf dem Sensorbildschirm 19 des mobilen Endgeräts 10 angezeigt. In Schritt 22 wird eine zu untersuchende Person angewiesen, das mobile Endgerät zum Nahpunkt hin zu bewegen. Diese Anweisung in Schritt 22 kann über eine Anzeige auf dem Sensorbildschirm 19 oder durch eine Sprachausgabe auf dem Lautsprecher 13 erfolgen.

Diese Bewegung zu dem Nahpunkt ist in Fig. 3A schematisch dargestellt. Hier hält eine Person 30 das mobile Endgerät 10 so, dass sie auf den Sensorbildschirm 19 blicken kann. Nach der Anweisung in Schritt 22 bewegt die Person 30 das mobile Endgerät 10 etwa wie durch einen Pfeil 33 angezeigt von einer größeren Entfernung auf das Auge zu, bis sie die Sehzeichen auf dem Sensorbildschirm 19 gerade noch scharf sehen kann. Alternativ oder bei wiederholtem Durchführen des Verfahrens auch zusätzlich kann die Person das mobile Endgerät 10 zuerst nahe bei Augen 32A, 32B (im Folgenden zusammenfassend als Augen 32 bezeichnet) halten und das mobile Endgerät 10 dann von den Augen weg bewegen, bis die Sehzeichen scharf gesehen werden können. Am Ende dieser Bewegung befindet sich das mobile Endgerät 10 im Nahpunkt, und in Schritt 23 der Fig. 2 empfängt das mobile Endgerät eine Eingabe von der Person 30, die bestätigt, dass der Nahpunkt erreicht ist. Diese Eingabe kann über den Sensorbildschirm 19 oder über den Lautsprecher 13 erfolgen.

Während dieser Bewegung entsprechend dem Pfeil 33 der Fig. 3 werden in Schritt 29 wiederholt Bilder mit der Rückkamera 12 des mobilen Endgeräts aufgenommen und Beschleunigungsdaten mittels des Beschleunigungssensors 17 aufgezeichnet. Die Rate, mit der diese Wiederholung stattfindet, ist im Wesentlichen durch die Rechenleistung des mobilen Endgeräts beschränkt, d.h. dadurch, wie schnell die aufgenommenen Bilder und gemessenen Beschleunigungen aufgenommen und verarbeitet werden können. Höhere Raten können dabei zu einer höheren Genauigkeit führen. In den aufgenommenen Bildern der Umgebung werden in Schritt 210 jeweils einander entsprechende Punkte, sogenannte Merkmalspunkte, identifiziert. Einander entsprechende Punkte sind dabei Punkte, die in jedem Bild den gleichen Teil des gleichen Objekts zeigen. Zur Veranschaulichung sind in der Fig. 3A verschiedene derartige Punkte 34A bis 34D gezeigt. Wie zu sehen ist, können diese Punkte Ecken von Möbelstücken (Punkte 34B, 34D), Punkte an einer Pflanze (Punkt 34C) oder Punkte an Bestandteilen von Räumen wie einer Balkonstange (Punkt 34A) sein. Üblicherweise werden hier Punkte verwendet, welche in ihrem lokalen Umfeld eine deutliche Struktur bezogen auf Farb- und/oder Helligkeitsunterschiede aufweisen. Diese sind relativ leicht mit herkömmlichen Verfahren der Bildbearbeitung identifizierbar. Einen Überblick über verschiedene hierzu verwendbare Verfahren gibt E. Salahat und M. Qasaimeh, "Recent advances in features extraction and description algorithms: A comprehensive survey", 2017 IEEE International Conference on Industrial Technology (ICIT), Toronto, ON, 2017, Seiten 1059-1063.

Aus den Beschleunigungsdaten und den aufgenommenen Bildern mit den darin identifizierten Punkten wird in Schritt 211 fortlaufend die Pose des mobilen Endgeräts 10, d.h. Position und Orientierung des mobilen Endgeräts, mittels visuell-inertialer Odometrie bestimmt. Wenn die Benutzereingabe in Schritt 23 empfangen wird, entspricht die Position des mobilen Endgeräts zu diesem Zeitpunkt der Position des Nahpunkts.

Die Position des mobilen Endgeräts wird dabei in einem Weltkoordinatensystem 35 der Fig. 3A berechnet. Der Ursprung dieses Weltkoordinatensystems kann wie bereits erläutert frei gewählt werden. Die Orientierung kann als Verkippung eines mit dem mobilen Endgerät verbundenen Koordinatensystems 36 gegenüber dem Weltkoordinatensystem 35 angegeben werden.

Zudem werden während der Bewegung zum Nahpunkt hin in Schritt 212 wiederholt, mittels der Frontkamera 19 des mobilen Endgeräts Bilder aufgenommen, auf welchen die Augen 32 der Person sichtbar sind, wie dies ebenfalls in Fig. 3A angedeutet ist. In Schritt 213 werden in diesen Bildern die Augen identifiziert. In Schritt 214 wird dann die Position der Augen basierend auf dem Identifizieren der Augen in den Frontkamerabildern und der jeweiligen Pose des mobilen Endgeräts (berechnet in Schritt 211) beim Aufnehmen der Frontkamerabilder berechnet. Die Position der Augen und die Position des mobilen Endgeräts am Nahpunkt werden dann in Schritt 24 als Koordinaten in dem Weltkoordinatensystem 35 gespeichert.

Das Berechnen der Position der Augen des Schritts 214 wird nunmehr unter Bezugnahme auf die Fig. 4 näher erläutert.

In Fig. 4 ist zur Erläuterung das mobile Endgerät 10 in drei verschiedenen Posen als mobiles Endgerät 10A, 10B bzw. 10C gezeigt. Das mit dem mobilen Endgerät gekoppelte Koordinatensystem 36 bewegt sich dabei mit dem mobilen Endgerät 10 und ist als Koordinatensystem 36A, 36B bzw. 36C in Fig. 4 dargestellt. Derartige mehrere Posen nimmt das mobile Endgerät 10 beispielsweise beim Bewegen zu dem Nahpunkt hin folgend der Anweisung in Schritt 22 an.

Während der Bewegung werden wie unter Bezugnahme auf die Fig. 2 erläutert Beschleunigungssensordaten aufgenommen und mittels der Rückkamera des mobilen Endgeräts 10A die Umgebung mit den Punkten aufgenommen (am Beispiel der Fig. 4 sind Punkte 34A, 34E, 34F und 34G dargestellt), sodass mittels visuell-inertialer Odometrie wie diskutiert die Pose des mobilen Endgeräts 10 zu jedem Punkt der Bewegung bestimmt werden kann. Die Pose wird dabei wie erwähnt als Position des mobilen Endgeräts in dem Weltkoordinatensystem 35 und Orientierung als Verkippung des Koordinatensystems 36 gegenüber dem Weltkoordinatensystem 35 angegeben werden.

Grundsätzlich sind für die Bestimmung der Position der Augen zwei Posen ausreichend. Da die Bewegung des mobilen Endgeräts 10 jedoch typischerweise in einer vergleichsweise geraden Linie auf die Augen zu erfolgt und somit der Unterschied zwischen den Posen quer zu dieser Richtung relativ gering zu einer Linie, die direkt auf die Augen weist, kann durch eine höhere Zahl von Posen die Genauigkeit erhöht werden. Vorzugsweise werden 2 bis 20 verschiedene Posen des mobilen Endgeräts 10 zur Bestimmung der Position der Augen der Person 30 verwendet. Die Darstellung von drei verschiedenen Posen in Fig. 4 ist dabei nur ein Beispiel. Grundsätzlich ist für eine derartige Positionsbestimmung ähnlich einer Triangulation nämlich ein größerer Abstand quer zu einer Linie auf das Objekt zu, dessen Position bestimmt werden soll (in diesem Fall der Augen), förderlich, was im vorliegenden Fall durch eine größere Anzahl von Posen und damit verbundene Mittelung ausgeglichen werden kann.

Die Position der Augen, beispielsweise der Pupillen der Augen oder von Augenwinkeln der Person 30, wird in den Bildern mit Verfahren der Bildverarbeitung detektiert. Ein von einer Kamera aufgenommenes digitales Bild besteht üblicherweise aus einer rechteckigen Anordnung von Bildpunkten (auch als Pixel bezeichnet, vom Englischen: picture elements). Die Positionen der Augen können dann als zweidimensionale Koordinaten in dieser rechteckigen Anordnung von Bildpunkten angegeben werden.

Die Berechnung, die nunmehr vorgestellt wird, gilt für ein Auge. Für das jeweils andere Auge kann die gleiche Berechnung durchgeführt werden, um so die Position beider Augen zu errechnen. Die Koordinaten des Auges in dem Bild werden in homogene Koordinatenschreibweise als

$$y_n = \begin{pmatrix} y_1 \\ y_2 \\ 1 \end{pmatrix} \qquad (1)$$

angegeben. Derartige homogene Koordinaten werden der projektiven Geometrie öfter verwendet, siehe Wikipedia Artikel "Homogene Koordinaten", Stand 4. Februar 2019. Der Index n von $y_n$ gibt dabei die Nummer der Pose wieder, d.h. beispielsweise für jede Pose des mobilen Endgeräts 10A, 10B, 10C liegt ein jeweiliger Wert $y_n$ vor.

Im Weltkoordinatensystem 35 hat das Auge hingegen die Position

$$x_{welt} = \begin{pmatrix} x_1 \\ x_2 \\ x_3 \\ 1 \end{pmatrix}, \qquad (2)$$

ebenfalls in homogener Schreibweise.

Der Zusammenhang zwischen $y_n$ und $x_{welt}$ ist gegeben durch

$$y_n = C \cdot X_n \cdot x_{welt} \qquad (3)$$

Dabei ist **C** die sogenannte Kameramatrix, welche die perspektivische Projektion auf einen zweidimensionalen Bildsensor der Kamera beschreibt. **C** ist für eine bestimmte Kamera festgelegt und kann für verschiedene Modelle von mobilen Endgeräten beispielsweise durch Kalibrierungsmessungen ermittelt werden oder kann vom Hersteller des mobilen Endgeräts bereitgestellt sein. Für eine einfache Lochkamera gilt

$$C = \begin{pmatrix} f & 0 & 0 & 0 \\ 0 & f & 0 & 0 \\ 0 & 0 & 1 & 0 \end{pmatrix} \qquad (4)$$

Dabei ist *f* die Brennweite der Lochkamera. **X** ist die Transformationsmatrix, welche die euklidische Koordinatentransformation von Punkten im Weltkoordinatensystem (35 in Fig. 4) zu dem jeweiligen Kamerakoordinatensystem entsprechend dem mit dem mobilen Endgerät 10 verbundenen Koordinatensystem 36 (36A, 36B und 36C in Fig. 4) beschreibt. **X** ist gegeben durch

$$X = \begin{pmatrix} R_n & t_n \\ 0 & 1 \end{pmatrix} \qquad (5)$$

$R_n$ ist die Rotationsmatrix, welche die Rotation des Koordinatensystems 36 gegenüber dem Koordinatensystem 35 bis schreibt (also die Orientierung), und $t_n$ ist ein Translationsvektor, welcher die jeweilige Position des mobilen Endgeräts 10, ausgedrückt als Verschiebung des Koordinatenursprung des Koordinatensystems 36 zu dem Koordinatenursprung des Koordinatensystems 35, zeigt.

Die Position kann dabei als Position eines bestimmten Punktes des mobilen Endgeräts 10 angegeben werden. In diesem Punkt des mobilen Endgerätes kann der Ursprung des Koordinatensystems 36 gelegt werden.

**X** ist also aus der Messung der Pose des mobilen Endgeräts mittels visuell-inertialer Odometrie bekannt. $y_n$ ist aus den Bildaufnahmen der Augen der Person mittels der Frontkamera 19 des mobilen Endgeräts 10 bekannt. **C** ist für ein jeweiliges mobiles Endgerät fest vorgegeben und beschreibt die Kameraeigenschaften. Somit sind aus Gleichung (3) nur die Koordinaten $x_{welt}$ des jeweiligen Auges in dem Weltkoordinatensystem unbekannt. Aus mehreren Messungen, d. h. mehrere Posen des mobilen Endgeräts wie in Fig. 4 gezeigt, kann das sich so ergebende Gleichungssystem mit Methoden der linearen Algebra und/oder numerischen Optimierung nach den Augenkoordinaten $x_{welt}$ aufgelöst werden.

Derartige Herangehensweisen sind in dem Artikel der englischsprachigen Wikipedia "Direct linear transformation", Stand 15. Februar 2019, und den dort zitierten Referenzen beschrieben.

Die so ermittelten Koordinaten der Augen werden wie bereits erwähnt in Schritt 24 der Fig. 2 abgespeichert.

Wenn die Positionen der Pupillen als Position der Augen verwendet werden, wird in Schritt 20 aus den Koordinaten der Augen optional der Pupillenabstand PD als Distanz zwischen den Koordinaten der Pupillen der Augen bestimmt.

In Schritt 25 wird die Nahpunktdistanz als Abstand zwischen dem Nahpunkt und den Augen bestimmt. Als Position des Nahpunkts werden dabei die in Schritt 24 abgespeicherten Koordinaten des mobilen Endgeräts am Nahpunkt verwendet. Als Position der Augen wird bei dem Verfahren der Fig. 4 die Mitte einer Verbindungslinie zwischen den Koordinaten der beiden Augen verwendet.

Die Nahpunktdistanz ist in Fig. 3A mit einem Pfeil 31 gekennzeichnet.

Auf diese Weise kann mit dem Verfahren der Fig. 2 die Nahpunktdistanz bestimmt werden. Weitergehende Verfahrensschritte, welche nun erläutert werden, dienen zum Bestimmen des sphärischen Brechwertes für eine Lesebrille.

Die nächsten Schritte des Verfahrens der Fig. 2 dienen zum Bestimmen der Lesedistanz. Wie eingangs erläutert kann stattdessen auch ein vorgegebener oder vom Benutzer eingegebener Wert für die Lesedistanz verwendet werden.

In Schritt 26 wird die Person angewiesen, das mobile Endgerät in eine Leseposition zu bewegen, d.h. in einer Lesedistanz zu halten. Wie bei Schritt 22 kann diese Anweisung durch eine Anzeige auf dem Sensorbildschirm 19 oder auch durch eine Ausgabe aus dem Lautsprecher 13 erfolgen.

In Schritt 218 wird von dem mobilen Endgerät eine Eingabe der Person empfangen, dass die Leseposition erreicht ist. Wie bei Schritt 23 kann dies beispielsweise durch entsprechendes Berühren des Sensorbildschirms oder durch eine Spracheingabe erfolgen.

Während der Bewegung in die Lesedistanz werden die Schritte 29, 110 und dann auch der Schritt 211 weiter durchgeführt. Zudem können auch die Schritte 212, 213 und 214 weiterhin durchgeführt werden, um die Position der Augen zu aktualisieren, wenn sich der Benutzer die Position der Augen seit dem Schritt 24 durch Bewegungen verändert. In Schritt 27 werden dann ähnlich dem Schritt 24 die Koordinaten des mobilen Endgeräts in der Leseposition und die Koordinaten der Augen gespeichert. Bei anderen Ausführungsbeispielen kann die Position der Augen, welche in Schritt 24 abgespeichert wurde, weiter verwendet werden. In diesem Fall sollte die Person zwischen den Schritten 24 und 27 die Position ihrer Augen möglichst nicht verändern.

Die Schritte 26, 218 und 27 sind in Fig. 3B veranschaulicht. In Fig. 3B hat die Person 30 das mobile Endgerät 10 in die Leseposition bewegt, d.h. in eine Position, in der sie typischerweise auf den Sensorbildschirm 19 des mobilen Endgeräts 10 blickt. Die Rückkamera nimmt weiter die Umgebung, insbesondere die Punkte 34A bis 34D auf, und bestimmt so zusammen mit Daten von dem Beschleunigungssensor 17 die Position des mobilen Endgeräts 10 in dem Weltkoordinatensystem 35.

In Schritt 28 der Fig. 2 wird dann aus den Schritt 27 abgespeicherten Koordinaten die Lesedistanz als Abstand zwischen dem mobilen Endgerät in der Leseposition und den Augen. Für den Augenabstand gilt dabei das Gleiche, wie bereits für Schritt 25 erläutert, d.h. als Position der Augen kann zum Zwecke der Abstandsbestimmung ein Mittelpunkt einer Strecke zwischen den Koordinaten des linken und rechten Auges verwendet werden.

Die Lesedistanz ist in Fig. 3B mit einem Pfeil 37 gekennzeichnet.

In Schritt 215 wird aus der in Schritt 25 bestimmten Nahpunktdistanz und aus der in Schritt 28 bestimmten Lesedistanz der sphärische Brechwert bestimmt, wie dies eingangs erläutert wurde.

In Schritt 216 wird der sphärische Brechwert dann mittels der Kommunikationsschaltung 18 des mobilen Endgeräts an einen Brillenglashersteller übertragen, wo entsprechende Brillengläser dann in Schritt 217 gefertigt werden.

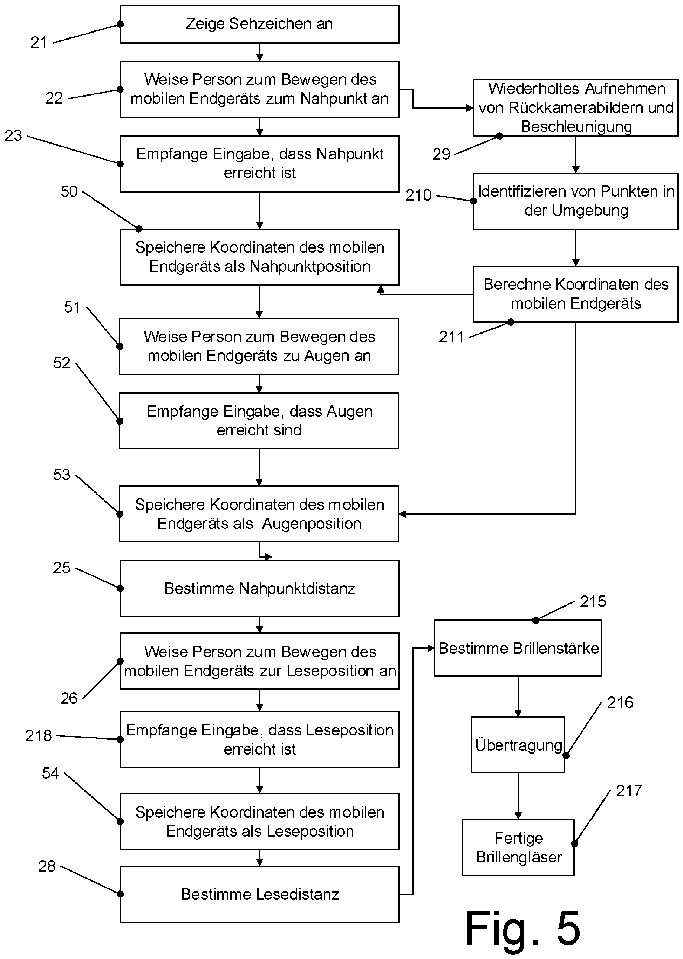

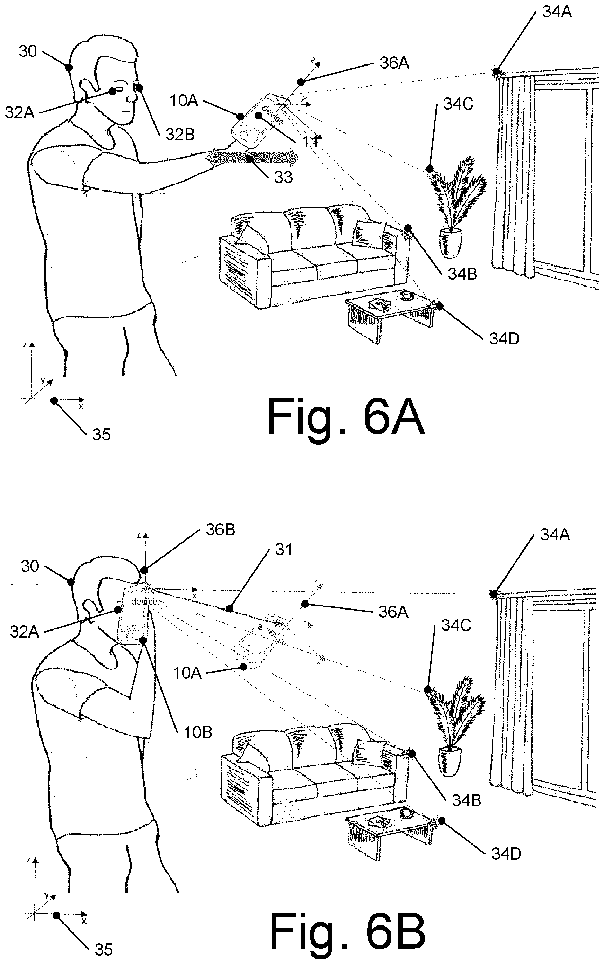

Ein weiteres Verfahren ist in Fig. 5 dargestellt. Zur Vermeidung von Wiederholungen wird bei der Beschreibung des Verfahrens der Fig. 5 auf die Beschreibung des Verfahrens der Fig. 2 Bezug genommen. Zudem werden die Fig. 6A bis 6C zur Veranschaulichung des Verfahrens der Fig. 5 herangezogen.

Schritte 21 bis 23 des Verfahrens der Fig. 5 entsprechen Schritten 21 bis 23 der Fig. 2, d.h. es werden Sehzeichen auf dem Sensorbildschirm 19 des mobilen Endgeräts 10 angezeigt, die Person wird angewiesen, das mobile Endgerät zum Nahpunkt zu bewegen, und das mobile Endgerät 10 empfängt eine Benutzereingabe, wenn der Nahpunkt erreicht ist. Parallel hierzu werden wie bei dem Verfahren der Fig. 2 die Schritte 29 bis 211 durchgeführt. Im Unterschied zu dem Schritt 211 der Fig. 2 muss in Schritt 211 der Fig. 5 nur die Position des mobilen Endgeräts berechnet werden und nicht die gesamte Pose einschließlich Orientierung, da für das Verfahren der Fig. 5 die Orientierung nicht benötigt wird.

In Schritt 50 werden dann die Koordinaten des mobilen Endgeräts in der Position, bei der die Eingabe der Person in Schritt 23 erhalten wird, als Nahpunktposition abgespeichert.

Die Schritte 21, 22, 23, 50 sowie die dabei ablaufenden Schritte 29, 210, 211 sind in Fig. 6A veranschaulicht. Die Fig. 6A entspricht dabei weitestgehend der Fig. 3A, wobei das mobile Endgerät am Nahpunkt hier mit dem Bezugszeichen 10A und das zugehörige Koordinatensystem mit dem Bezugszeichen 36A gekennzeichnet sind, um sie von späteren, im weiteren Verlauf des Verfahrens verwendeten Positionen zu unterscheiden.

In Schritt 51 wird die Person dann angewiesen, das mobile Endgerät zu den Augen zu bewegen. Die Anweisung kann wie bei Schritt 22 über eine Ausgabe auf dem Sensorbildschirm oder eine Ausgabe aus dem Lautsprecher 13 erfolgen.

In Schritt 52 wird eine Benutzereingabe empfangen, dass die Augen erreicht sind. Während der Bewegung zu den Augen laufen wiederum die Schritte 29, 210 und 211 ab, und die Koordinaten der Position des mobilen Endgeräts beim Empfangen der Eingabe in Schritt 52 werden in Schritt 53 als Augenposition abgespeichert. Die Schritte 51 und 52 und die dazu parallel ablaufenden Schritte 29 bis 211 sind in Fig. 6B dargestellt. Hier hat die Person 30 das mobile Endgerät zu den Augen bewegt. In diese Position ist das mobile Endgerät mit dem Bezugszeichen 10B bezeichnet.

In Schritt 25 wird dann die Nahpunktdistanz als Abstand zwischen den in Schritt 50 gespeicherten Koordinaten und den in Schritt 53 gespeicherten Koordinaten bestimmt. In manchen Fällen kann ein gewisser Versatz, beispielsweise 0,5 cm oder 1 cm, zu der als Differenz der Koordinaten bestimmten Nahpunktdistanz hinzuaddiert werden, um die Nahpunktdistanz zu bestimmen, um zu berücksichtigen, dass aufgrund der Anatomie des Gesichts das mobile Endgerät gegebenenfalls noch einen gewissen Abstand zu den Augen hat.

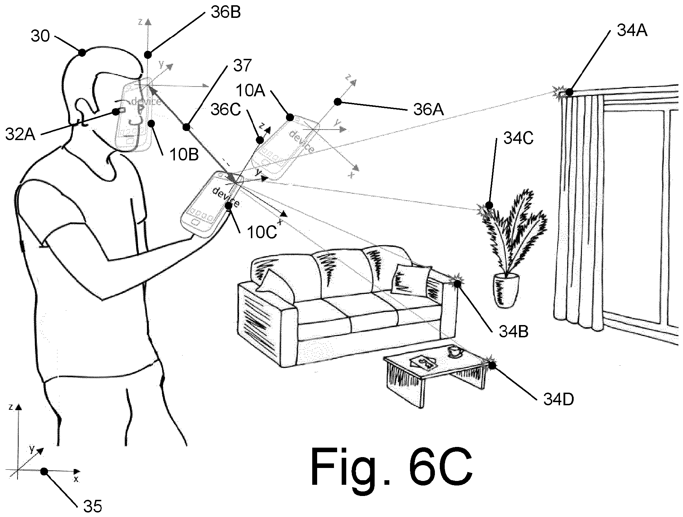

Die Schritte 26 und 218 entsprechen wiederum den Schritten 26 und 218 der Fig. 2, d.h. hier wird der Benutzer angewiesen, das mobile Endgerät in die Leseposition zu bewegen, und nach Erreichen der Leseposition bei 218 empfängt das mobile Endgerät eine Benutzereingabe. Währenddessen laufen wieder die Schritte 29 bis 211 ab, und in Schritt 54 werden die Koordinaten des mobilen Endgeräts beim Empfangen der Benutzereingabe von Schritt 218 als Leseposition abgespeichert. Dies ist in Fig. 6C veranschaulicht, wo die Person 30 das mobile Endgerät 10 in der Leseposition hält, in der das mobile Endgerät mit dem Bezugszeichen 10C gekennzeichnet ist. In Schritt 28 wird die Lesedistanz dann als Differenz zwischen den in den Schritten 53 und 54 gespeicherten Koordinaten berechnet, wobei auch hier wiederum ein Versatz hinzuaddiert werden kann, um einen Abstand des mobilen Endgeräts von den Augen, wenn das mobile Endgerät direkt vor die Augen an den Kopf gehalten wird, zu berücksichtigen.

Die Lesedistanz ist in Fig. 6C mit einem Pfeil 37 gekennzeichnet.

Die Schritte 215 bis 217 des Verfahrens der Fig. 5 entsprechen wiederum den Schritten 215 bis 217 der Fig. 2. Verglichen mit der Fig. 2 benötigt das Verfahren der Fig. 5 keine Bestimmung der Augenpositionen mittels Triangulation, dafür muss die Person 30 das mobile Endgerät noch in eine dritte Position, nämlich in eine Position möglichst nahe bei den Augen, bewegen.

Die Verfahren der Fig. 2 und 5 können insbesondere durch ein entsprechendes Programm, welches in dem Speicher 15 des mobilen Endgeräts abgelegt ist und auf dem Prozessor 16 läuft, implementiert werden.

Manche Ausführungsbeispiele werden durch die folgenden Klauseln definiert:

Klausel 1. Computerimplementiertes Verfahren zum Bestimmen des Nahpunkts (31) einer Person (30) mit einem mobilen Endgerät, umfassend:

Bestimmen der Position des mobilen Endgeräts (10) an dem Nahpunkt,
dadurch gekennzeichnet,
dass das Bestimmen der Position des mobilen Endgeräts an dem Nahpunkt auf Basis wiederholter Aufnahmen einer Umgebung des mobilen Endgeräts (10) mit einer eingebauten Kamera (11, 12) des mobilen Endgeräts (10) und auf Basis von wiederholtem Messen einer Beschleunigung des mobilen Endgeräts (10) mit einem eingebauten Beschleunigungssensor (17) des mobilen Endgeräts während einer Bewegung des mobilen Endgeräts (10) zum Nahpunkt erfolgt.

Klausel 2. Verfahren nach Klausel 1, gekennzeichnet durch:
Anzeigen von Sehzeichen auf dem mobilen Endgerät (10).

Klausel 3. Verfahren nach Klausel 1 oder 2, gekennzeichnet durch:

Auffordern der Person (30), das mobile Endgerät (10) zu dem Nahpunkt der Person zu bewegen,
Erhalten einer Rückmeldung, wenn das mobile Endgerät an dem Nahpunkt der Person (30) ist.

Klausel 4. Verfahren nach einer der Klauseln 1 bis 3, dadurch gekennzeichnet, dass die wiederholten Aufnahmen mit einer Rückkamera des mobilen Endgeräts (10) erfolgen, welche auf einer Anzeige (19) des mobilen Endgeräts (10) gegenüberliegenden Seite des mobilen Endgeräts angeordnet ist.

Klausel 5. Computerimplementiertes Verfahren zum Bestimmen der Nahpunktdistanz (31) einer Person (30) mit einem mobilen Endgerät (10), gekennzeichnet durch:

Bestimmen des Nahpunkts der Person mit dem Verfahren nach einer der Klauseln 1 bis 4, Bestimmen der Position von mindestens einem Auge (32A, 32B) der Person (30), und
Bestimmen der Nahpunktdistanz (31) auf Basis der Position des mobilen Endgeräts (10) an dem Nahpunkt und der Position des mindestens einen Auges (32A, 32B).

Klausel 6. Verfahren nach Klausel 5, dadurch gekennzeichnet, dass das Bestimmen der Position des mindestens einen Auges umfasst:

während der Bewegung des mobilen Endgeräts (10) zum Nahpunkt der Person,
wiederholtes Bestimmen von Position und Orientierung des mobilen Endgeräts (10) auf Basis der wiederholten Aufnahmen der Umgebung und dem wiederholten Messen der Beschleunigungwiederholtes Aufnehmen eines Bilds des mindestens einen Auges der Person mit der eingebauten Kamera (12) oder einer weiteren eingebauten Kamera (11) des mobilen Endgeräts (10),
Identifizieren des mindestens einen Auges in den Bildern des mindestens einen Auges der Person (30), und
Bestimmen der Position des mindestens einen Auges (32A, 32B) der Person auf Basis der Positionen und Orientierungen des mobilen Endgeräts und den identifizierten Positionen der Augen in den Bildern.

Klausel 7. Verfahren nach Klausel 6, wobei die Position des mindestens einen Auges die Positionen beider Augen der Person umfasst, wobei die Positionen der Augen Positionen der Pupillen der Augen umfassen, und wobei das Verfahren weiter ein Bestimmen des Pupillenabstands auf Basis der Positionen der Pupillen umfasst.

Klausel 8. Verfahren nach Klausel 6 oder 7, wobei die Position des mindestens einen Auges die Positionen beider Augen der Person umfasst und wobei das Bestimmen der Nahpunktdistanz ein Bestimmen der Nahpunktdistanz als Abstand zwischen der Position des mobilen Endgeräts an dem Nahpunkt und einem Mittelpunkt einer Verbindungslinie zwischen den Positionen der Augen umfasst.

Klausel 9. Verfahren nach einer der Klauseln 6 bis 8, wobei das wiederholte Aufnehmen des Bilds des mindestens eines Auges mit einer Frontkamera (11) des mobilen Endgeräts (10) erfolgt, welche auf einer gleichen Seite des mobilen Endgeräts (10) wie ein Bildschirm (19) des mobilen Endgeräts angeordnet ist.

Klausel 10. Verfahren nach Klausel 5, dadurch gekennzeichnet, dass das Bestimmen der Position des mindestens einen Auges der Person umfasst:
Bestimmen der Position des mobilen Endgeräts an den Augen der Person auf Basis von wiederholtem Aufnehmen der Umgebung des mobilen Endgeräts (10) mit der eingebauten Kamera (12) des mobilen Endgeräts und wiederholtem Messen der Beschleunigung des mobilen Endgeräts (10) mit dem eingebauten Beschleunigungssensor (17) des mobilen Endgeräts während einer Bewegung des mobilen Endgeräts (10) zu den Augen mit dem eingebauten Beschleunigungssensor (17) des mobilen Endgeräts der Person.

Klausel 11. Verfahren nach Klausel 10, gekennzeichnet durch:

Auffordern der Person, das mobile Endgerät zu den Augen (32A, 32B) der Person (30) zu bewegen,
Erhalten einer Rückmeldung, wenn das mobile Endgerät (10) an den Augen (32A, 32B) der Person ist,.

Klausel 12. Verfahren zum Bestimmen eines sphärischen Brechwertes für ein Brillenglas, umfassend:

Bestimmen der Nahpunktdistanz (31) der Person (30) mit dem Verfahren nach einer der Klauseln 5 bis 11, und
Bestimmen des sphärischen Brechwertes auf Basis der Nahpunktdistanz (31) und einer Lesedistanz (37) der Person.

Klausel 13. Verfahren nach Klausel 12, wobei die Lesedistanz eine vorgegebene Lesedistanz ist, oder wobei die Lesedistanz von der Person (30) eingegeben wird.

Klausel 14. Verfahren nach Klausel 12, dadurch gekennzeichnet, dass das Verfahren weiter umfasst:

Bestimmen der Position des mobilen Endgeräts (10) in der Leseposition auf Basis von wiederholtem Aufnehmen der Umgebung des mobilen Endgeräts (10) mit der eingebauten Kamera (12) des mobilen Endgeräts und wiederholtem Messen der Beschleunigung des mobilen Endgeräts (10) mit dem eingebauten Beschleunigungssensor (17) des mobilen Endgeräts während einer Bewegung des mobilen Endgeräts in die Leseposition, und
Bestimmen der Lesedistanz auf Basis der Position des mobilen Endgeräts (10) in der Leseposition und der Position der Augen.

Klausel 15. Verfahren nach Klausel 14, gekennzeichnet durch:

Auffordern der Person, das mobile Endgerät (10) in eine Leseposition der Person (30) zu bewegen, Erhalten einer Rückmeldung, wenn das mobile Endgerät (10) in der Leseposition ist,

Klausel 16. Verfahren zum Herstellen eines Brillenglases, umfassend:

Bestimmen des sphärischen Brechwertes nach einer der Klauseln 12 bis 15, und Herstellen eines Brillenglases auf Basis der bestimmten sphärischen Brechwertes.

Klausel 17. Computerprogramm für ein mobiles Endgerät mit einer Kamera (11, 12) und einem Beschleunigungssensor (17), umfassend Befehle, die bei der Ausführung des Computerprogramms durch ein mobiles Endgerät (10) dieses veranlassen, das Verfahren nach einem der Klauseln 1 bis 15 auszuführen.

Klausel 18. Computerlesbarer nichtflüchtiger Datenträger, auf dem das Computerprogramm nach Klausel 17 gespeichert ist.

Klausel 19. Datenträgersignal, das das Computerprogramm nach Klausel 17 überträgt.

Klausel 20. Mobiles Endgerät, umfassend:

eine eingebaute Kamera (11, 12), einen Beschleunigungssensor (17), und einen Prozessor (16), der so konfiguriert ist, dass folgende Schritte ausgeführt werden:

Bestimmen der Position des mobilen Endgeräts (10) an dem Nahpunkt, dadurch gekennzeichnet, dass das Bestimmen der Position des mobilen Endgeräts an dem Nahpunkt auf Basis wiederholter Aufnahmen einer Umgebung des mobilen Endgeräts (10) mit einer eingebauten Kamera (11, 12) des mobilen Endgeräts (10) und auf Basis von wiederholtem Messen einer Beschleunigung des mobilen Endgeräts (10) mit einem eingebauten Beschleunigungssensor (17) des mobilen Endgeräts während einer Bewegung des mobilen Endgeräts (10) zum Nahpunkt erfolgt.

Klausel 21. Mobiles Endgerät nach Klausel 20, dadurch gekennzeichnet, dass der Prozessor so konfiguriert ist, dass das Verfahren nach einem der Klauseln 1 bis 15 ausgeführt wird.

**Patentansprüche**

1. Computerimplementiertes Verfahren zum Bestimmen des Nahpunkts (31) einer Person (30) mit einem mobilen Endgerät, umfassend:

Bestimmen der Position des mobilen Endgeräts (10) an dem Nahpunkt, **dadurch gekennzeichnet,** **dass** das Bestimmen der Position des mobilen Endgeräts an dem Nahpunkt auf Basis wiederholter Aufnahmen einer Umgebung des mobilen Endgeräts (10) mit einer eingebauten Kamera (11, 12) des mobilen Endgeräts (10) und auf Basis von wiederholtem Messen einer Beschleunigung des mobilen Endgeräts (10) mit einem eingebauten Beschleunigungssensor (17) des mobilen Endgeräts während einer Bewegung des mobilen Endgeräts (10) zum Nahpunkt erfolgt.

2. Verfahren nach Anspruch 1, **gekennzeichnet durch**:

Auffordern der Person (30), das mobile Endgerät (10) zu dem Nahpunkt der Person zu bewegen, Erhalten einer Rückmeldung, wenn das mobile Endgerät an dem Nahpunkt der Person (30) ist.

3. Verfahren nach 1 oder 2, **dadurch gekennzeichnet, dass** die wiederholten Aufnahmen mit einer Rückkamera des mobilen Endgeräts (10) erfolgen, welche auf einer Anzeige (19) des mobilen Endgeräts (10) gegenüberliegenden Seite des mobilen Endgeräts angeordnet ist.

4. Computerimplementiertes Verfahren zum Bestimmen der Nahpunktdistanz (31) einer Person (30) mit einem mobilen Endgerät (10), **gekennzeichnet durch**:

 Bestimmen des Nahpunkts der Person mit dem Verfahren nach einem der Ansprüche 1 bis 3,
 Bestimmen der Position von mindestens einem Auge (32A, 32B) der Person (30), und
 Bestimmen der Nahpunktdistanz (31) auf Basis der Position des mobilen Endgeräts (10) an dem Nahpunkt und der Position des mindestens einen Auges (32A, 32B).

5. Verfahren nach Anspruch 4, **dadurch gekennzeichnet, dass** das Bestimmen der Position des mindestens einen Auges umfasst:

 während der Bewegung des mobilen Endgeräts (10) zum Nahpunkt der Person,
 wiederholtes Bestimmen von Position und Orientierung des mobilen Endgeräts (10) auf Basis der wiederholten Aufnahmen der Umgebung und dem wiederholten Messen der Beschleunigung wiederholtes Aufnehmen eines Bilds des mindestens einen Auges der Person mit der eingebauten Kamera (12) oder einer weiteren eingebauten Kamera (11) des mobilen Endgeräts (10),
 Identifizieren des mindestens einen Auges in den Bildern des mindestens einen Auges der Person (30), und
 Bestimmen der Position des mindestens einen Auges (32A, 32B) der Person auf Basis der Positionen und Orientierungen des mobilen Endgeräts und den identifizierten Positionen der Augen in den Bildern.

6. Verfahren nach Anspruch 5, wobei die Position des mindestens einen Auges die Positionen beider Augen der Person umfasst und wobei das Bestimmen der Nahpunktdistanz ein Bestimmen der Nahpunktdistanz als Abstand zwischen der Position des mobilen Endgeräts an dem Nahpunkt und einem Mittelpunkt einer Verbindungslinie zwischen den Positionen der Augen umfasst.

7. Verfahren nach Anspruch 5 oder 6, wobei das wiederholte Aufnehmen des Bilds des mindestens einen Auges mit einer Frontkamera (11) des mobilen Endgeräts (10) erfolgt, welche auf einer gleichen Seite des mobilen Endgeräts (10) wie ein Bildschirm (19) des mobilen Endgeräts angeordnet ist.

8. Verfahren nach Anspruch 4, **dadurch gekennzeichnet, dass** das Bestimmen der Position des mindestens einen Auges der Person umfasst:
 Bestimmen der Position des mobilen Endgeräts an den Augen der Person auf Basis von wiederholtem Aufnehmen der Umgebung des mobilen Endgeräts (10) mit der eingebauten Kamera (12) des mobilen Endgeräts und wiederholtem Messen der Beschleunigung des mobilen Endgeräts (10) mit dem eingebauten Beschleunigungssensor (17) des mobilen Endgeräts während einer Bewegung des mobilen Endgeräts (10) zu den Augen mit dem eingebauten Beschleunigungssensor (17) des mobilen Endgeräts der Person.

9. Verfahren nach Anspruch 8, **gekennzeichnet durch**:

 Auffordern der Person, das mobile Endgerät zu den Augen (32A, 32B) der Person (30) zu bewegen,
 Erhalten einer Rückmeldung, wenn das mobile Endgerät (10) an den Augen (32A, 32B) der Person ist.

10. Verfahren zum Bestimmen eines sphärischen Brechwertes für ein Brillenglas, umfassend:

 Bestimmen der Nahpunktdistanz (31) der Person (30) mit dem Verfahren nach einem der Ansprüche 4 bis 9, und
 Bestimmen des sphärischen Brechwertes auf Basis der Nahpunktdistanz (31) und einer Lesedistanz (37) der Person.

11. Verfahren nach Anspruch 10, **dadurch gekennzeichnet, dass** das Verfahren weiter umfasst:

 Bestimmen der Position des mobilen Endgeräts (10) in der Leseposition auf Basis von wiederholtem Aufnehmen der Umgebung des mobilen Endgeräts (10) mit der eingebauten Kamera (12) des mobilen Endgeräts und wiederholtem Messen der Beschleunigung des mobilen Endgeräts (10) mit dem eingebauten Beschleunigungssensor (17) des mobilen Endgeräts während einer Bewegung des mobilen Endgeräts in die Leseposition, und
 Bestimmen der Lesedistanz auf Basis der Position des mobilen Endgeräts (10) in der Leseposition und der Position der Augen.

**12.** Verfahren nach Anspruch 11, **gekennzeichnet durch**:

Auffordern der Person, das mobile Endgerät (10) in eine Leseposition der Person (30) zu bewegen,
Erhalten einer Rückmeldung, wenn das mobile Endgerät (10) in der Leseposition ist.

**13.** Verfahren zum Herstellen eines Brillenglases, umfassend:

Bestimmen des sphärischen Brechwertes nach einem der Ansprüche 10 bis 12, und
Herstellen eines Brillenglases auf Basis der bestimmten sphärischen Brechwertes.

**14.** Computerprogramm für ein mobiles Endgerät mit einer Kamera (11, 12) und einem Beschleunigungssensor (17), umfassend Befehle, die bei der Ausführung des Computerprogramms durch ein mobiles Endgerät (10) dieses veranlassen, das Verfahren nach einem der Ansprüche 1 bis 12 auszuführen.

**15.** Mobiles Endgerät, umfassend:

eine eingebaute Kamera (11, 12),
einen Beschleunigungssensor (17), und
einen Prozessor (16), der so konfiguriert ist, dass folgende Schritte ausgeführt werden:

Bestimmen der Position des mobilen Endgeräts (10) an dem Nahpunkt,
**dadurch gekennzeichnet,**
**dass** das Bestimmen der Position des mobilen Endgeräts an dem Nahpunkt auf Basis wiederholter Aufnahmen einer Umgebung des mobilen Endgeräts (10) mit einer eingebauten Kamera (11, 12) des mobilen Endgeräts (10) und auf Basis von wiederholtem Messen einer Beschleunigung des mobilen Endgeräts (10) mit einem eingebauten Beschleunigungssensor (17) des mobilen Endgeräts während einer Bewegung des mobilen Endgeräts (10) zum Nahpunkt erfolgt.

11   13   12

Lautsprecher

14   Mikrofon

19

Sensorbildschirm

10

15   16   17   18

Speicher   Prozessor   Beschl.-
sensor   TX/RX

# Fig. 1

17

2/7

Zeige Sehzeichen an — 21

Weise Person zum Bewegen des mobilen Endgeräts zum Nahpunkt an — 22

Empfange Eingabe, dass Nahpunkt erreicht ist — 23

Speichere Koordinaten des mobilen Endgeräts und der Augen — 24

Bestimme Pupillenabstand — 20

Bestimme Nahpunktdistanz — 25

Weise Person zum Bewegen des mobilen Endgeräts zur Leseposition an — 26

Empfange Eingabe, dass Leseposition erreicht ist — 218

Speichere Koordinaten des mobilen Endgeräts und der Augen — 27

Bestimme Lesedistanz — 28

Wiederholtes Aufnehmen von Frontkamerabildern — 212

Identifiziere Augen in Frontkamerabildern — 213

Berechne Augenkoordinaten — 214

Wiederholtes Aufnehmen von Rückkamerabildern und Beschleunigung — 29

Identifizieren von Punkten in der Umgebung — 210

Berechne Pose des mobilen Endgeräts — 211

Bestimme sphärische Brechkraft — 215

Übertragung — 216

Fertige Brillengläser — 217

# Fig. 2

18

Fig. 3A

Fig. 3B

Fig. 4

```
┌─────────────────────────────┐
│      Zeige Sehzeichen an     │
└─────────────────────────────┘
              │
              ▼
┌─────────────────────────────┐
│  Weise Person zum Bewegen des│              ┌──────────────────────────┐
│  mobilen Endgeräts zum       │─────────────▶│  Wiederholtes Aufnehmen  │
│  Nahpunkt an                 │              │ von Rückkamerabildern und│
└─────────────────────────────┘              │      Beschleunigung      │
              │                               └──────────────────────────┘
              ▼                                           │
┌─────────────────────────────┐                          ▼
│  Empfange Eingabe, dass      │              ┌──────────────────────────┐
│  Nahpunkt erreicht ist       │              │  Identifizieren von      │
└─────────────────────────────┘              │  Punkten in der Umgebung │
              │                               └──────────────────────────┘
              ▼                                           │
┌─────────────────────────────┐                          ▼
│  Speichere Koordinaten des   │              ┌──────────────────────────┐
│  mobilen Endgeräts als       │◀─────────────│  Berechne Koordinaten des│
│  Nahpunktposition            │              │      mobilen Endgeräts   │
└─────────────────────────────┘              └──────────────────────────┘
              │
              ▼
┌─────────────────────────────┐
│  Weise Person zum Bewegen des│
│  mobilen Endgeräts zu Augen an│
└─────────────────────────────┘
              │
              ▼
┌─────────────────────────────┐
│  Empfange Eingabe, dass      │
│  Augen erreicht sind         │
└─────────────────────────────┘
              │
              ▼
┌─────────────────────────────┐
│  Speichere Koordinaten des   │◀─────────────────────────┐
│  mobilen Endgeräts als       │                          │
│  Augenposition               │                          │
└─────────────────────────────┘                          │
              │
              ▼
┌─────────────────────────────┐
│  Bestimme Nahpunktdistanz    │
└─────────────────────────────┘
              │
              ▼                               ┌──────────────────────────┐
┌─────────────────────────────┐              │  Bestimme Brillenstärke  │
│  Weise Person zum Bewegen des│─────────────▶└──────────────────────────┘
│  mobilen Endgeräts zur       │                          │
│  Leseposition an             │                          ▼
└─────────────────────────────┘              ┌──────────────────────────┐
              │                               │       Übertragung        │
              ▼                               └──────────────────────────┘
┌─────────────────────────────┐                          │
│  Empfange Eingabe, dass      │                          ▼
│  Leseposition erreicht ist   │              ┌──────────────────────────┐
└─────────────────────────────┘              │   Fertige Brillengläser  │
              │                               └──────────────────────────┘
              ▼
┌─────────────────────────────┐
│  Speichere Koordinaten des   │
│  mobilen Endgeräts als       │
│  Leseposition                │
└─────────────────────────────┘
              │
              ▼
┌─────────────────────────────┐
│  Bestimme Lesedistanz        │
└─────────────────────────────┘
```

# Fig. 5

Fig. 6A

Fig. 6B

Fig. 6C

**Europäisches Patentamt**
**European Patent Office**
**Office européen des brevets**

# EUROPÄISCHER RECHERCHENBERICHT

Nummer der Anmeldung

EP 20 18 8386

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/116499 A1 (SINGER MICHAEL E [US] ET AL) 3. Mai 2018 (2018-05-03) | 1-4,8-15 | INV. A61B3/10 |
| A | * Absätze [0007], [0021], [0024], [0027], [0035], [0037], [0042] * | 5-7 | |
| A,D | US 2016/120402 A1 (LIMON OFER [IL]) 5. Mai 2016 (2016-05-05) * Absätze [0122], [0125], [0138], [0141] * | 1-15 | |
| A | US 2017/202450 A1 (CARRAFA JOSEPH [US] ET AL) 20. Juli 2017 (2017-07-20) * Absätze [0025] - [0027] * | 1-15 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| München | 13. Januar 2021 | Martelli, Luca |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 20 18 8386

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2021

| Im Recherchenbericht angeführtes Patentdokument | Datum der Veröffentlichung | Mitglied(er) der Patentfamilie | Datum der Veröffentlichung |
|---|---|---|---|
| US 2018116499 A1 | 03-05-2018 | US 2018116499 A1 | 03-05-2018 |
| | | US 2019274540 A1 | 12-09-2019 |
| | | WO 2018081704 A1 | 03-05-2018 |
| US 2016120402 A1 | 05-05-2016 | AU 2014276340 A1 | 24-12-2015 |
| | | BR 112015030406 A2 | 25-07-2017 |
| | | CA 2914456 A1 | 11-12-2014 |
| | | CN 105764405 A | 13-07-2016 |
| | | CN 110251066 A | 20-09-2019 |
| | | EP 3003121 A1 | 13-04-2016 |
| | | IL 242949 A | 28-11-2019 |
| | | JP 6453859 B2 | 16-01-2019 |
| | | JP 6612959 B2 | 27-11-2019 |
| | | JP 2016523132 A | 08-08-2016 |
| | | JP 2019069180 A | 09-05-2019 |
| | | KR 20160017079 A | 15-02-2016 |
| | | RU 2015152290 A | 14-07-2017 |
| | | US 2016120402 A1 | 05-05-2016 |
| | | US 2017079523 A1 | 23-03-2017 |
| | | US 2019307324 A1 | 10-10-2019 |
| | | WO 2014195951 A1 | 11-12-2014 |
| US 2017202450 A1 | 20-07-2017 | CA 3011428 A1 | 20-07-2017 |
| | | EP 3402384 A1 | 21-11-2018 |
| | | JP 2019501743 A | 24-01-2019 |
| | | US 2017202450 A1 | 20-07-2017 |
| | | WO 2017123523 A1 | 20-07-2017 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160120402 A1
- WO 2018002332 A2

**In der Beschreibung aufgeführte Nicht-Patentliteratur**

- **H. HOFSTETTER.** Useful age-amplitude formula. *Optom World,* 1950, vol. 38 (12), 42-45
- **A. DUANE.** Normal values of the accommodation at all ages. *J. Am. Med. Assoc.,* 1912, vol. 59, 1010-1013
- **C. I. BRAUN ; W. E. BENSON ; N. A. REMALEY ; E. Y. CHEW ; F. L. FERRIS.** Accommodative amplitudes in the Early Treatment Diabetic Retinopathy Study. *Retina,* 1995, vol. 15 (4), 275-81
- **B. GILMARTIN.** The Marton Lecture: Ocular Manifestations Of Systemic Medication. *Ophthalmic and Physiological Optics,* 1987, vol. 7 (4), 449-459
- Dynamic Skiametry and Methods of Testing the Accommodation and Convergence of the Eyes. **C. SHEARD.** Physiological optics. Cleveland Press, 1920, 108
- **F. DONDERS ; W. MOORE.** On the anomalies of accommodation and refraction of the eye with a preliminary essay on physiological dioptrics. The New Sydenham Society
- **IDE, TAKESHI et al.** The Effect of 3D Visual Simulator on Children's Visual Acuity - A Pilot Study Comparing Two Different Modalities. *The Open Ophthalmology Journal,* 2013, vol. 7, 69-48
- *PMC, Web,* 11. April 2018
- **IDE, TAKESHI ; NEGISHI, KAZUNO ; YAMAGUCHI, TAKEFUMI ; HARA, SHUYA ; TODA, IKUKO ; TSUBOTA, KAZUO.** New Compact Accommodometer to Measure Accommodation Amplitude as a Biomarker. *The Asia-Pacific Journal of Ophthalmology,* 2012, 24-27
- Technological enhancements to optometric clinical tests. **ALEC KINGSNORTH.** Dissertation. Aston University, Marz 2015
- **G. NÜTZLI et al.** Fusion of IMU and Vision for absolute scale estimation in monocular SLAM. *J. Intel. Robot Syst.,* 2011, vol. 61, 287-299
- **F. TIMM ; E. BARTH.** Accurate eye centre localisation by means of gradients. *Proceedings of the Int. Conference on Computer Theory and Applications (VISAPP),* 2011, vol. 1, 125-130
- **E. SALAHAT ; M. QASAIMEH.** Recent advances in features extraction and description algorithms: A comprehensive survey. *2017 IEEE International Conference on Industrial Technology (ICIT),* 2017, 1059-1063
- Homogene Koordinaten. *Wikipedia Artikel,* Februar 2019
- Artikel der englischsprachigen Wikipedia. *Direct linear transformation,* Februar 2019